(19) Europäisches Patentamt
European Patent Office
Office européen des brevets

(11) Numéro de publication : **0 591 026 A1**

# (12) DEMANDE DE BREVET EUROPEEN

(21) Numéro de dépôt : **93402280.7**

(22) Date de dépôt : **20.09.93**

(51) Int. Cl.$^5$ : **C07D 401/14,** A61K 31/445

(30) Priorité : **28.09.92 FR 9211550**

(43) Date de publication de la demande :
**06.04.94 Bulletin 94/14**

(84) Etats contractants désignés :
**AT BE CH DE DK ES FR GB GR IE IT LI LU MC NL PT SE**

(71) Demandeur : **SYNTHELABO**
**22, Avenue Galilée**
**F-92350 Le Plessis Robinson (FR)**

(72) Inventeur : **Jegham, Samir**
**65 rue du Lieutenant Colonel Prudhon**
**F-95100 Argenteuil (FR)**
Inventeur : **Defosse, Gérard**
**29 rue de Tolbiac**
**F-75013 Paris (FR)**
Inventeur : **Purcell, Thomas**
**47 bis rue de la Millière, Les Mesnuls**
**F-78490 Montfort l'Amaury (FR)**

(74) Mandataire : **Thouret-Lemaitre, Elisabeth et al**
**SYNTHELABO, Service Brevets, B.P. 72**
**F-92352 Le Plessis-Robinson Cédex (FR)**

(54) **Dérivés de pipéridine, leur préparation et leur application en thérapeutique.**

(57) Composés répondant à la formule (I)

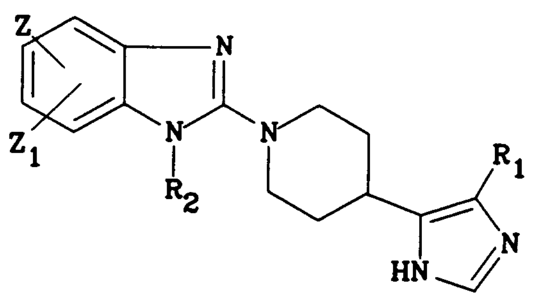

(I)

dans laquelle
$R_1$ représente soit un atome d'hydrogène, soit un groupe ($C_1$-$C_6$)alkyle droit ou ramifié,
$R_2$ représente soit un atome d'hydrogène, soit un groupe ($C_1$-$C_8$)alkyle droit ou ramifié,
Z et $Z_1$ représentent, chacun indépendamment l'un de l'autre, soit un atome d'hydrogène, soit un atome de chlore, soit un groupe hydroxy, soit un groupe amino, soit un groupe nitro, soit un groupe hydroxyméthyle, soit un groupe ($C_1$-$C_2$)alkyle, soit un groupe ($C_1$-$C_8$)alcoxy, soit un groupe ($C_1$-$C_5$)alcoxycarbonyle droit ou ramifié, soit un groupe aryl($C_1$-$C_2$)alcoxy Z est en position 4, 6 ou 7 et Z et $Z_1$ ne peuvent représenter simultanément un atome d'hydrogène,
ainsi que leurs sels d'addition aux acides pharmaceutiquement acceptables.
Application thérapeutique.

EP 0 591 026 A1

La présente invention a pour objet des dérivés de pipéridine, leur préparation et leur application en thérapeutique.

Les composés de l'invention répondent à la formule (I)

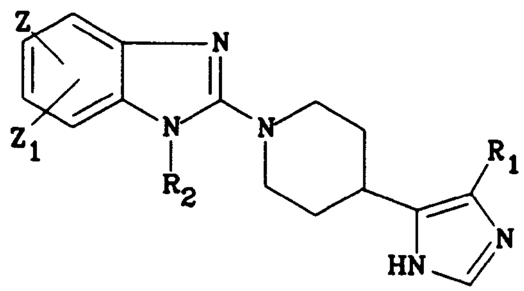

(I)

dans laquelle

$R_1$ représente soit un atome d'hydrogène, soit un groupe $(C_1\text{-}C_6)$alkyle droit ou ramifié,

$R_2$ représente soit un atome d'hydrogène, soit un groupe $(C_1\text{-}C_8)$alkyle droit ou ramifié,

Z et $Z_1$ représentent, chacun indépendamment l'un de l'autre, soit un atome d'hydrogène, soit un atome de chlore, soit un groupe hydroxy, soit un groupe amino, soit un groupe nitro, soit un groupe hydroxyméthyle, soit un groupe $(C_1\text{-}C_2)$alkyle, soit un groupe $(C_1\text{-}C_8)$alcoxy, soit un groupe $(C_1\text{-}C_5)$alcoxycarbonyle droit ou ramifié, soit un groupe aryl$(C_1\text{-}C_2)$alcoxy,

Z est en position 4, 6 ou 7 et Z et $Z_1$ ne peuvent représenter simultanément un atome d'hydrogène.

Selon l'invention, les composés préférés sont les composés de formule (I) dans laquelle Z est en position 7.

Parmi ces composés, les composés particulièrement préférés sont ceux où $Z_1$ représente un atome d'hydrogène.

Ils peuvent se présenter à l'état de bases libres ou de sels d'addition aux acides pharmaceutiquement acceptables. Les composés dont la formule est une forme mésomère de la formule (I), font partie de l'invention.

Conformément à l'invention, on peut préparer les composés de formule (I) selon le procédé illustré dans le schéma 1.

**Schéma 1**

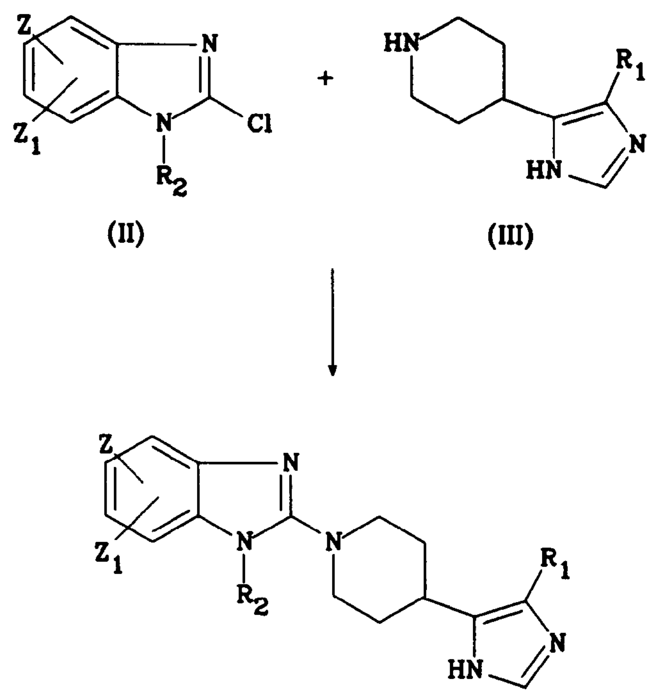

(II) (III)

(I)

On fait réagir un dérivé de benzimidazole de formule (II) dans laquelle $R_2$, Z et $Z_1$ sont tels que définis précédemment avec un dérivé de pipéridine de formule (III) dans laquelle $R_1$ est tel que défini précédemment. La réaction est réalisée dans un solvant tel que, par exemple, l'alcool isopentylique. Les composés de formule (I) dans laquelle $Z_1$ représente un groupe amino sont obtenus à partir des composés de formule (I) dans laquelle $Z_1$ représente un groupe nitro, selon des techniques décrites dans la littérature ou connues de l'homme du métier.

Les composés de formule (I) dans laquelle Z ou $Z_1$ représente un groupe hydroxy sont obtenus respectivement à partir des composés de formule (I) dans laquelle Z ou $Z_1$ représente un groupe méthoxy, selon des techniques décrites dans la littérature ou connues de l'homme du métier.

Les composés de formule (I) dans laquelle Z ou $Z_1$ représente un groupe hydroxyméthyle sont obtenus respectivement à partir des composés de formule (I) dans laquelle Z ou $Z_1$ représente un groupe alcoxycarbonyle, selon des techniques décrites dans la littérature ou connues de l'homme du métier.

Les composés de départ sont disponibles dans le commerce ou décrits dans la littérature ou peuvent être préparés selon des méthodes qui y sont décrites ou qui sont connues de l'homme du métier.

Le 2-chloro-3-nitrophénol est préparé selon la technique décrite dans *J. Prakt. Chem.*, 1930, **127**, 20.

Le 2-chloro-4(7)-méthoxybenzimidazole est préparé selon la technique décrite dans *Chemical Abstract* 67 : 108597x.

Le 2-[[(1,1-diméthyléthoxy)carbonyl]amino]-3-nitrobenzoate d'éthyle est préparé selon la technique décrite dans le brevet européen 0459136.

La 4-chloro-5-nitro-benzène-1,2-diamine est décrite dans *Heterocycle,* 1987, **26**, n° 9, 2433.

Les composés de formule (II) ont été préparés suivant des protocoles voisins de ceux décrits dans *J. Med. Chem.,* 1986, **29**, 1178-83 et dans le brevet européen n° 0039190.

La 4-(1*H*-imidazol-4-yl)pipéridine est décrite dans *Arch. Pharmaz.,* (Weinheim. Ger.) 1973, **306**(12), 934-42 et dans la demande de brevet européen 0197840.

La 4-(5-méthyl-1*H*-imidazol-4-yl)pyridine est décrite dans *J. Med. Chem.*, 1986, **29**, 2154-63.

Les exemples 1 à 4 qui suivent illustrent la préparation de quelques composés de formule (II).

Les exemples 5 à 9 qui suivent illustrent en détail la préparation des composés selon l'invention.

Les microanalyses et les spectres IR et RMN confirment la structure des composés obtenus.

Exemple 1

2-chloro-1-(1-méthyléthyl)-7-phénylméthoxy-1*H*-benzimidazole

1.1. 2-chloro-3-nitrophénol

On dissout à chaud 73 g (0,52 mole) de 3-nitrophénol dans 2 litres d'acide chlorhydrique 6 N. On refroidit au bain de glace jusqu'à 28-30 °C. On ajoute en 1,5 heures 32 g (0,26 mole) de chlorate de potassium dans 500 ml d'eau, puis, à la fin de l'addition, on laisse sous agitation pendant 30 minutes à 28-30 °C. On refroidit à 15 °C et on ajoute 1 litre de dichlorométhane. On décante et on lave à l'eau. On sèche la phase organique et on évapore. On purifie le résidu par chromatographie sur colonne de gel de silice en éluant par un mélange de dichlorométhane/méthanol (80/20). On recristallise dans le toluène.
On obtient 29 g de produit.
Point de fusion = 130 °C Rendement = 32 %

1.2. 2-chloro-1-nitro-3-(phénylméthoxy)benzène

Dans un ballon tricol de 250 ml, on place 1,43 g (0,036 mole) d'une solution d'hydrure de sodium à 60 % dans 25 ml de diméthylformamide. A la température ambiante on ajoute 6 g du dérivé chloré obtenu précédemment en 1.1 puis on ajoute goutte à goutte 6,6 g (0,039 mole) de (bromométhyl)benzène. On laisse sous agitation pendant 24 heures à la température ambiante. On jette le milieu réactionnel sur un mélange d'eau glacée et d'hexane, on essore, on lave dans un mélange d'eau glacée et d'hexane puis on sèche, à 60 °C, sous vide.
On obtient 8,4 g de produit.
Point de fusion = 94 °C Rendement = 92 %

1.3. *N*-(1-méthyléthyl)-2-nitro-6-(phénylméthoxy)benzèneamine

On place 8,4 g (0,032 mole) du dérivé chloré obtenu précédemment en 1.2 dans 20 ml d'isopropylamine et on chauffe au bain d'huile dans une autoclave, à 110 °C pendant 18 heures. On évapore l'amine en excès et on reprend par un mélange d'eau et d'éther. On recueille la phase organique, on la lave à l'eau, on la sèche et on évapore.
On obtient 8,2 g de produit.
Rendement = 90 %

1.4. $N^2$-(1-methyléthyl)-3-(phénylméthoxy)benzène-1,2-diamine

Dans une fiole de Parr de 250 ml, on place 100 mg (0,44 mole) d'oxyde de platine dans 25 ml d'éthanol. On hydrogène pendant 15 minutes, sous une pression de 15 psi puis on ajoute 0,5 g (0,0017 mole) du dérivé nitré obtenu précédemment en 1.3. On laisse pendant 5 minutes, on filtre le catalyseur et on évapore le solvant. On reprend le résidu par de l'éther et on le lave successivement par de la soude 1 N et par de l'eau. On sèche et on évapore.
Rendement = 92 %

1.5. 1-(1-méthyléthyl)-7-(phénylméthoxy)-1,3-dihydro-2*H*-benzimidazol-2-one

On chauffe à 175-180 °C pendant 2 heures, un mélange de 2,2 g (0,036 mole) d'urée et 6,6 g (0,026 mole) de la diamine précédemment obtenue en 1.4. On reprend le résidu par un mélange d'eau et d'éther puis on évapore. On reprend le dernier résidu gommeux par de l'éther et on filtre.
On obtient 4,6 g de produit.
Point de fusion = 224 °C Rendement = 64 %

1.6. 2-chloro-1-(1-méthyléthyl)-7-phénylméthoxy-1*H*-benzimidazole

On chauffe au reflux pendant 1,5 heures 4 g (0,014 mole) du composé précédemment obtenu en 1.5, dans 50 ml de chlorure de phosphoryle. On évapore le solvant, on refroidit à 0 °C et on alcalinise par un mélange eau/ammoniaque jusqu'à pH 8. On extrait par un mélange éther/dichlorométhane, on sèche et on évapore. On purifie le résidu par chromatographie sur colonne de gel de silice en éluant par un mélange méthanol/dichlorométhane (2/98).
On obtient 2 g de produit.
Rendement = 48 %

Exemple 2

2-chloro-4(7)-méthoxy-1-(1-méthyléthyl)-1*H*-benzimidazole

***Voie 1***

A un mélange de 5,3 g (0,029 mole) de 2-chloro-4(7)-méthoxy-1*H*-benzimidazole dans 30 ml de diméthylsulfoxyde et de 4,41 g (0,032 mole) de carbonate de potassium, on ajoute 3,74 g (0,031 mole) de 2-bromopropane, puis on chauffe pendant 3 heures à 60 °C. On verse le milieu réactionnel sur de l'eau, on extrait par de l'éther et on lave successivement par de l'eau et par une solution saturée en chlorure de sodium. On sèche, on filtre et on évapore. On purifie le brut obtenu par chromatographie sur colonne de gel de silice en éluant par un mélange cyclohexane/acétate d'éthyle (4/1).
On obtient 2,5 g de 2-chloro-4-méthoxy-1-(1-méthyléthyl)-1*H*-benzimidazole (A) et 2,5 g de 2-chloro-7-méthoxy-1-(1-méthyléthyl)-1*H*-benzimidazole (B).
A : Point de fusion = 99-101 °C Rendement = 40 %
B : Point de fusion = 63 °C Rendement = 40 %

***Voie 2***

On prépare le 2-chloro-7-méthoxy-1-(1-méthyléthyl)-1*H*-benzimidazole selon la méthode décrite dans l'exemple 1 à partir du 2-chloro-1-méthoxy-3-nitrobenzène.
Point de fusion = 63 °C Rendement = 44 %

Exemple 3

2-chloro-1-(1-méthyléthyl)-1*H*-benzimidazole-7-carboxylate d'éthyle

3.1. 2-[(1-méthyléthyl)amino]-3-nitrobenzoate d'éthyle

A une solution de 34 g (0,11 mole) de 2[[(1,1-diméthyléthoxy)carbonyl]amino]-3-nitrobenzoate d'éthyle dans 100 ml de diméthylformamide, on ajoute, à 0 °C, sous argon, par portions, 4,8 g (0,12 mole) d'une solution d'hydrure de sodium à 60 %. On laisse sous agitation pendant 20 minutes à 0 °C. On ajoute alors 55,9 g (0,33 mole) de 2-iodopropane et on chauffe à 80 °C pendant 4 heures puis à la température ambiante pendant 48 heures. On hydrolyse en ajoutant de l'eau et on extrait 3 fois par de l'acétate d'éthyle. On recueille la phase organique, on la lave successivement par de l'eau et par une solution saturée en chlorure de sodium. On sèche et on évapore.

3.2. 3-amino-2-[(1-méthyléthyl)amino]benzoate d'éthyle

On hydrogène à 45 °C, pendant 5 heures, sous pression ordinaire, une solution de 27 g (0,011 mole) du dérivé nitré précédemment obtenu en 3.1, dans 250 ml d'acétate d'éthyle, en présence de 2,7 g de palladium sur charbon à 10 %. On filtre le catalyseur, on évapore le solvant et on purifie le résidu par chromatographie sur colonne de gel de silice en éluant par un mélange hexane/acétate d'éthyle (9/1). On obtient le produit sous forme d'une huile.

3.3. 1-(1-méthyléthyl)-2-oxo-2,3-dihydro-1*H*-benzimidazole-7-carboxylate d'éthyle

On chauffe à 180 °C pendant 8 heures, un mélange de 5 g (0,023 mole) de la diamine obtenue précédemment en 3.2 et 4 g (0,066 mole) d'urée. On reprend le résidu par de l'éther et on filtre.
Rendement = 60 %

3.4. 2-chloro-1-(1-méthyléthyl)-1*H*-benzimidazole-7-carboxylate d'éthyle

On chauffe au reflux pendant 20 heures, 3,2 g (0,013 mole) de la benzimidazolone précédemment obtenue

en 3.3, dans 25 ml de chlorure de phosphoryle. On évapore le chlorure de phosphoryle, on alcalinise à pH 8 avec de l'ammoniaque et on extrait par de l'acétate d'éthyle. On recueille la phase organique, on évapore et on sèche. On purifie par chromatographie sur colonne de gel de silice en éluant par un mélange dichlorométhane/méthanol (95/5).

Exemple 4

2,5-dichloro-6-nitro-1*H*-benzimidazole

4.1. 5-chloro-6-nitro-1,3-dihydro-2*H*-benzimidazol-2-one

On chauffe à 180 °C pendant 4 heures un mélange de 14 g (0,074 mole) de 4-chloro-5-nitro-benzène-1,2-diamine et de 13,44 g (0,226 mole) d'urée. On refroidit le milieu réactionnel et on évapore à sec. On reprend le résidu par du méthanol et on évapore à nouveau. On triture le résidu dans l'éther et on obtient un solide que l'on filtre. On utilise le produit tel quel dans les étapes ultérieures.

4.2. 2,5-dichloro-6-nitro-1*H*-benzimidazole

On le prépare selon la méthode décrite dans l'exemple 1.6 à partir de la benzimidazolone précédemment obtenue en 4.1.
Rendement = 32 %

Exemple 5

2-[4-(1*H*-imidazol-4-yl)pipéridin-1-yl]-1-(1-méthyléthyl)-7-(phénylméthoxy)-1*H*-benzimidazole, maléate

On chauffe pendant 48 heures à 120 °C un mélange de 1 g (0,0066 mole) de 4-(1*H*-imidazol-4-yl)pipéridine et de 1 g (0,0033 mole) de dérivé chloré préparé selon la méthode décrite dans l'exemple 1.6, dans 4 ml d'alcool isopentylique. On évapore le solvant et on reprend le résidu par un mélange d'eau et de dichlorométhane. On décante, on recueille la phase organique, on évapore et on sèche. On purifie le résidu par chromatographie sur colonne de gel de silice en éluant par un mélange dichlorométhane/méthanol/ammoniaque (45/5/0,5).
On obtient 0,9 g de produit pur.
Rendement = 73 %
On prépare le maléate dans l'acétone. On dissout le produit sous forme de base dans l'acétone, on ajoute un équivalent d'une solution alcoolique d'acide maléique, on agite, on évapore et on recristallise le produit, sous forme de maléate, dans l'acétone.
Point de fusion = 158-159 °C

Exemple 6

2-[4-(1*H*-imidazol-4-yl)pipéridin-1-yl]-7-méthoxy-1-(1-méthyléthyl)-1*H*-benzimidazole

On chauffe pendant 96 heures à 120 °C un mélange de 1,34 g (0,009 mole) de 4-(1*H*-imidazol-4-yl)pipéridine et de 1 g (0,0045 mole) de dérivé chloré préparé selon la méthode décrite dans l'exemple 2, dans 4 ml d'alcool isopentylique. On évapore le solvant et on reprend le résidu par un mélange d'eau et d'éther. On décante, on recueille la phase organique, on la lave à l'eau et à l'éther, on évapore et on sèche. On purifie le résidu par chromatographie sur colonne de gel de silice en éluant par un mélange dichlorométhane/méthanol/ammoniaque (45/5/0,5). On obtient 1 g de produit pur que l'on recristallise dans un mélange contenant du dichlorométhane, du méthanol et de l'éther.
On obtient 0,9 g de produit.
Point de fusion = 250-253 °C Rendement = 60 %

Exemple 7

2-[4-(1*H*-imidazol-4-yl)pipéridin-1-yl]-1-(1-méthyléthyl)-1*H*-benzimidazole-7-carboxylate d'éthyle

On fait réagir le dérivé chloré précédemment obtenu en 3.4 avec de la 4-(1*H*-imidazol-4-yl)pipéridine selon la méthode décrite précédemment dans l'exemple 5.
On prépare le fumarate dans l'éthanol en ajoutant un équivalent d'une solution d'acide fumarique. Après agitation et évaporation, on recristallise le fumarate dans l'éthanol.
Point de fusion = 163-165 °C Rendement = 53 %

Exemple 8

7-hydroxyméthyl-2-[4-(1*H*-imidazol-4-yl)pipéridin-1-yl)-1-(1-méthyléthyl)-1*H*-benzimidazole

A une solution de 0,6 g (0,002 mole) d'ester, préparé selon la méthode décrite dans l'exemple 7, dans 10 ml de tétrahydrofurane, à 0 °C, on ajoute 10 ml d'une solution d'hydrure de diisobutyle d'aluminium 1 M dans le tétrahydrofurane. On laisse remonter, sous agitation, à la température ambiante et on maintient l'agitation pendant 24 heures. On ajoute un mélange de tétrahydrofurane et d'eau, on filtre, on lave par du dichlorométhane et on évapore le solvant. On purifie le résidu par chromatographie sur colonne de gel de silice en éluant par un mélange dichlorométhane/méthanol (8/2).

On prépare le fumarate comme décrit dans l'exemple 7.

Point de fusion = 186-193 °C (déc) Rendement = 45 %

Exemple 9

2-[4-(5-méthyl-1*H*-imidazol-4-yl)pipéridin-1-yl]-5-chloro-6-nitro-1*H*-benzimidazole

On fait réagir le dérivé chloré obtenu selon la méthode décrite dans l'exemple 4.2 avec la 4-(5-méthyl-1*H*-imidazol-4-yl)pipéridine dans les conditions décrites dans l'exemple 5.

Point de fusion = > 250 °C Rendement = 40 %

Le tableau suivant illustre les structures chimiques et les propriétés physiques de quelques composés selon l'invention.

***Légende du tableau***

**dans la colonne "F (°C)" du tableau**

**(dec) signifie décomposition**

**dans la colonne "Sel" du tableau**

**(x:y) signifie x moles d'acide pour y moles de base,**

**l'absence de toute mention signifie que le composé est à l'état de base,**

**chlor. représente le chlorhydrate,**

**fum. représente le fumarate,**

**mal. représente le maléate.**

Tableau

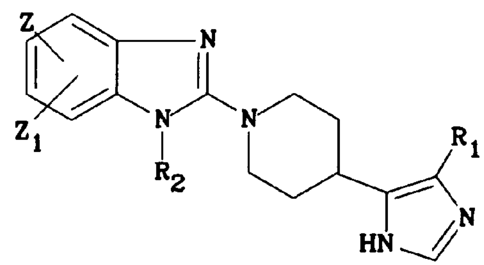

(I)

| N° | $R_1$ | $R_2$ | Z | $Z_1$ | F (°C) | Sel |
|---|---|---|---|---|---|---|
| 1 | -H | $-CH(CH_3)_2$ | 7-Cl | -H | 195-197 | fum. (1:1) |
| 2 | -H | $-CH(CH_3)_2$ | 7-OH | -H | 174-180 | - |
| 3 | -H | $-CH(CH_3)_2$ | 4-OH | -H | 264-265 | - |
| 4 | -H | $-CH(CH_3)_2$ | $7-CH_2OH$ | -H | 186-193 (dec) | fum. (1:1) |
| 5 | -H | $-CH(CH_3)_2$ | $7-CH_3$ | -H | 178-180 | fum. (1:1) |
| 6 | -H | $-CH(CH_3)_2$ | $4-CH_3$ | -H | 180-185 | fum. (1:1) |

| N° | $R_1$ | $R_2$ | Z | $Z_1$ | F (°C) | Sel |
|---|---|---|---|---|---|---|
| 7 | -H | $-CH(CH_3)_2$ | $4-OCH_3$ | -H | 105-110 | - |
| 8 | -H | $-CH(CH_3)_2$ | $7-OCH_3$ | -H | 250-253 | - |
| 9 | -H | $-CH(CH_3)_2$ | $7-OC_8H_{17}$ | -H | 147 | mal. (2:1) |
| 10 | -H | $-CH(CH_3)_2$ | $7-OCH_2C_6H_5$ | -H | 158-159 | mal. (2:1) |
| 11 | -H | $-CH(CH_3)_2$ | $7-COOC_2H_5$ | -H | 163-165 | fum. (1:1) |
| 12 | $-CH_3$ | $-CH(CH_3)_2$ | 7-Cl | -H | 192-195 | fum. (1:1) |
| 13 | $-CH_3$ | $-CH(CH_3)_2$ | $4-OCH_3$ | -H | 111-119 | - |
| 14 | $-CH_3$ | $-CH(CH_3)_2$ | $7-OCH_3$ | -H | 273-275 | - |
| 15 | $-CH_3$ | $-CH(CH_3)_2$ | $7-OC_8H_{17}$ | -H | 136-138 | - |
| 16 | $-CH_3$ | $-CH(CH_3)_2$ | $7-COO(CH_2)_2CH(CH_3)_2$ | -H | 188-190 | fum. (1:1) |
| 17 | $-CH_3$ | $-CH(CH_3)_2$ | $7-OCH_2C_6H_5$ | -H | 170-172 | fum. (3:2) |
| 18 | $-CH_3$ | -H | $6-NO_2$ | 5-Cl | > 250 | - |
| 19 | $-CH_3$ | -H | $6-NH_2$ | 5-Cl | > 300 | chlor. (2:1) |

Les composés de l'invention ont fait l'objet d'essais pharmacologiques qui ont montré leur intérêt comme substances actives en thérapeutique.

Ainsi ils ont été testés quant à leurs effets inhibiteurs de la liaison de la $[^3H]$quipazine avec les récepteurs sérotoninergiques de type $5-HT_3$ présents dans le cortex cérébral du rat, selon une variante de la méthode décrite par Milburn et Peroutka (*J. Neurochem.*, **52**, 1787-1792, 1989).

On utilise des rats mâles Sprague-Dawley, de 150 à 200 g, dans tous les essais. On en prélève le cortex cérébral et on l'homogénéise dans 20 volumes (poids/volume) de tampon Hepes 25 mM ou de tampon Hepes 25 mM contenant du chlorure de sodium (180 mM), du chlorure de calcium (2,5 mM), du chlorure de potassium

(5 mM) et du chlorure de magnésium (1,2 mM) (pH=7,4), à l'aide d'un broyeur Polytron™. Après centrifugation de la suspension pendant 10mn à 45000xg, on remet le culot en suspension dans le volume initial de tampon, contenant éventuellement 0,05% de Triton X-100™, et on effectue une première incubation de 30mn à 37°C. On effectue ensuite encore deux centrifugations comme précédemment décrit, et on reprend le culot final dans 11,7 volumes de tampon Hepes 25 mM à pH=7,4.

On détermine la liaison de la [$^3$H]quipazine (51,6-69,8 Ci/mmole, New England Nuclear, Boston, Ma, USA) en faisant incuber 150 μl de la suspension membranaire avec le radioligand (0,8 nM) dans un volume final de 1 ml, pendant 30 min à 25°C, en absence ou en présence du composé à étudier. L'incubation a lieu en présence de 0,1 μM de paroxétine et de 1 μM de kétansérine. La liaison non spécifique est déterminée en présence de 1 μM d'ondansetron. Après l'incubation, on dilue le mélange testé avec 5 ml de tampon Tris-HCl glacé 50 mM (pH=7,4 à 0°C), On collecte les membranes par filtration sur des filtres Whatman GF/B™ prétraités avec 0,05% de polyéthylèneimine, et on les lave avec trois volumes de 5 ml de tampon Tris-HCl glacé 50 mM.

La radioactivité retenue sur les filtres est mesurée par spectrométrie de scintillation liquide à une efficacité de 50 à 60%.

Les résultats s'expriment par la concentration ($CI_{50}$) de composé étudié qui inhibe 50% de la liaison de la [$^3$H]quipazine, déterminée par une méthode graphique ou mathématique. Les composés de l'invention les plus actifs dans cet essai se caractérisent par des $CI_{50}$ inférieures à 1 nM ($10^{-9}$M).

Les composés de l'invention ont été également testés quant à leur effet sur le réflexe de Bezold-Jarisch, c'est-à-dire une intense bradycardie, provoqué par injection intraveineuse de sérotonine. Ce reflexe met en jeu la stimulation des récepteurs spécifiques 5-$HT_3$ du nerf vague, ce qui provoque une dépolarisation et donc une sécrétion d'acétylcholine, qui est le neurotransmetteur vagal naturel.

On anesthésie des rats mâles Sprague-Dawley à l'uréthane (1 à 25 g/kg par voie intrapéritonéale), on mesure la pression sanguine grâce à un cathéter placé dans l'artère carotide, et on se sert des impulsions de pression pour activer un cardiotachymètre. Des canules sont placées dans les deux veines fémorales pour faciliter l'administration intraveineuse des produits.

On trace les courbes doses/réponses de la bradycardie provoquée par injection de doses de 30 μg/kg de sérotonine, avant et après l'injection des composés à étudier.

Les composés de l'invention les plus actifs dans cet essai inhibent la bradycardie provoquée par la sérotonine d'au moins 50% à une dose de 10 μg/kg administrée par voie intraveineuse.

Les composés de l'invention ont également été étudiés quant à leurs effets sur l'émèse chez le furet (mâle, 1 à 1,4 kg), selon une méthode décrite par Costall *et al.* (*Neuropharma-cology* **25**(8), 959-961, 1986).

Les composés à étudier ou du sérum physiologique sont administrés par voie intraveineuse (veine jugulaire), sous anesthésie à l'halothane, immédiatement avant une perfusion intraveineuse de cisplatine (10 mg/kg en 10 min).

On observe ensuite les animaux pendant 3h, en notant le nombre de crises émétiques, le nombre total de spasmes et de vomissements, ainsi que le délai d'apparition de la première crise.

Les composés de l'invention les plus actifs dans cet essai montrent un effet anti-émétique après administration d'une dose inférieure à 5 mg/kg par voie orale.

Enfin les composés de l'invention ont été étudiés quant à leurs effets sur les récepteurs 5-HT atypiques (5-$HT_4$) dans l'iléon de cobaye, d'après Craig et Clarke, *J. Pharm. Exp. Ther.*, **252** (3), 1378-1386, (1990).

Des cobayes tricolores Jegard mâles de 300 à 400 g sont assommés et saignés. On prélève rapidement un fragment d'iléon de 3 cm environ, au niveau de la jonction iléo-coecale, et on le lave avec 10 ml de tampon Krebs tiédi (composition en mM : NaCl=118 ; $CaCl_2$=2,6 ; KCl=4,9 ; $NaH_2PO_4$=1 ; $MgSO_4$=1,2 ; NaH-$CO_3$=25 ; Glucose=11,7). L'iléon est monté sur une pipette de 2 ml et le muscle longitudinal est séparé soigneusement avec un coton dentaire imbibé de tampon Krebs. L'organe est connecté à un transducteur isométrique sous une tension basale de 0,5 g et maintenu dans un bain de Krebs à 37°C aéré par un courant carbogène. Après un repos d'environ 30mn on applique une stimulation électrique (0,2 Hz ; 1,5 ms ; tension supra-maximale ≦45 V) grace à des électrodes de champ H. Sachs, modèle F2H reliées à un stimulateur Grass S88™, jusqu'à stabilisation des contractions (ou "twitches"). On ajoute alors dans le bain $3 \cdot 10^{-7}$M de phénoxybenzamine, ce qui diminue l'amplitude des contractions jusqu'à environ 50% (≦30 min). L'organe est ensuite lavé six fois à 5 min d'intervalle.

Avant le quatrième lavage on ajoute de la sérotonine ($3 \cdot 10^{-7}$M). Si nécessaire on diminue l'amplitude des contractions à 50% de l'amplitude supramaximale par diminution de la tension électrique après le sixième lavage. On construit une courbe concentration-effet par additions cumulatives, à 1 min d'intervalle, du composé à étudier.

Les réponses sont mesurées en terme de capacité à restaurer l'amplitude des contractions au niveau de celle obtenue grâce à la tension supramaximale et après traitement avec la phénoxybenzamine.

Les composés de l'invention se comportent comme des agonistes, agonistes partiels ou antagonistes desdits

récepteurs, certains d'entre eux étant actifs à des concentrations inférieures à 10 nM.

Les résultats des essais biologiques montrent que les composés de l'invention sont des ligands des récepteurs sérotoninergiques. Comme montré ci-dessus, ils ont en particulier une interaction avec les récepteurs de types 5-$HT_3$ et 5-$HT_4$.

Ils peuvent donc être utilisés pour le traitement et la prévention des désordres dans lesquels les récepteurs sérotoninergiques sont impliqués, tels que nausées et vomissements, par exemple consécutifs à un traitement antitumoral ou à l'administration d'un anesthésique ; troubles du système nerveux central tels que la schizophrénie, la manie, l'anxiété et la dépression ; troubles de la cognition tels que la démence sénile ou présénile d'Alzheimer ; dyskinésie, douleurs, migraines et maux de tête ; troubles de la dépendance ou du sevrage d'alcool ou de drogues ; troubles de la fonction gastrointestinale tels que dyspepsie, ulcère peptique, aigreurs d'estomac, flatulences ; troubles du système cardiovasculaire et troubles respiratoires.

A cet effet ils peuvent être présentés sous toutes formes appropriées à l'administration orale ou parentérale, telles que comprimés, dragées, gélules, capsules, suspensions ou solutions buvables ou injectables, etc. en association avec des excipients convenables, et dosées pour permettre une administration de 0,005 à 5 mg/kg de 1 à 4 fois par jour.

## Revendications

1. Composés répondant à la formule (I)

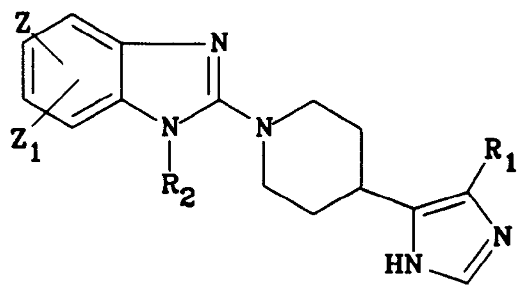

(I)

dans laquelle
$R_1$ représente soit un atome d'hydrogène, soit un groupe ($C_1$-$C_6$)alkyle droit ou ramifié,
$R_2$ représente soit un atome d'hydrogène, soit un groupe ($C_1$-$C_8$)alkyle droit ou ramifié,
Z et $Z_1$ représentent, chacun indépendamment l'un de l'autre, soit un atome d'hydrogène, soit un atome de chlore, soit un groupe hydroxy, soit un groupe amino, soit un groupe nitro, soit un groupe hydroxyméthyle, soit un groupe ($C_1$-$C_2$)alkyle, soit un groupe ($C_1$-$C_8$)alcoxy, soit un groupe ($C_1$-$C_5$)alcoxycarbonyle droit ou ramifié, soit un groupe aryl($C_1$-$C_2$)alcoxy,
Z est en position 4, 6 ou 7 et Z et $Z_1$ ne peuvent représenter simultanément un atome d'hydrogène, ainsi que leurs sels d'addition aux acides pharmaceutiquement acceptables.

2. Composés selon la revendication 1, caractérisés en ce que Z est en position 7.

3. Composés selon la revendication 2, caractérisés en ce que $Z_1$ représente un atome d'hydrogène.

4. Procédé de préparation des composés selon la revendication 1, procédé caractérisé en ce que l'on fait réagir un dérivé de benzimidazole de formule (II)

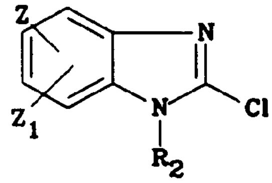

(II)

dans laquelle $R_2$, Z et $Z_1$ sont tels que définis dans la revendication 1, avec un dérivé de pipéridine de

formule (III)

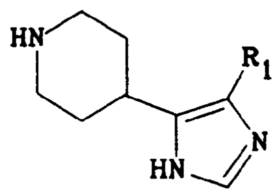

(III)

dans laquelle $R_1$ est tel que défini dans la revendication 1.

5. Médicament caractérisé en ce qu'il contient un composé selon l'une quelconque des revendications 1 à 3.

6. Composition pharmaceutique caractérisée en ce qu'elle contient un composé selon l'une quelconque des revendications 1 à 3, en association avec tout excipient approprié.

Office européen des brevets

# RAPPORT DE RECHERCHE EUROPEENNE

Numero de la demande
EP 93 40 2280

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| A | EP-A-0 304 353 (SYNTHELABO)<br>* le document en entier *<br>--- | 1-6 | C07D401/14<br>A61K31/445 |
| A | EP-A-0 445 026 (ADIR ET COMPAGNIE)<br>* revendications *<br>--- | 1-6 | |
| A | EP-A-0 379 990 (ISTITUTO LUSO FARMACO D'ITALIA S.P.A.)<br>* le document en entier *<br>--- | 1-6 | |
| P,X | EP-A-0 507 650 (SYNTHELABO)<br>* le document en entier *<br>----- | 1-6 | |

| DOMAINES TECHNIQUES RECHERCHES (Int.Cl.5) |
|---|
| C07D<br>A61K |

Le présent rapport a été établi pour toutes les revendications

| Lieu de la recherche | Date d'achèvement de la recherche | Examinateur |
|---|---|---|
| LA HAYE | 21 Décembre 1993 | Chouly, J |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)